# EUROPEAN PATENT APPLICATION

(11) **EP 0 951 899 A1**
(43) Date of publication of application: **27.10.1999**
(21) Application number: 99400645.0
(22) Date of filing: 16.03.1999
(51) Int. Cl.: A61K 7/06, C08G 77/388, C08G 77/26

(54) **Hair-care treatment composition**

(30) Priority: 01.04.1998 JP 8876098
(71) Applicant: SHIN-ETSU CHEMICAL CO., LTD., Chiyoda-ku Tokyo (JP)
(72) Inventor: Kuwata, Satoshi, c/o Shin-Etsu Chemical Co., Ltd., Usui-gun, Gunma-ken (JP); Hirai, Motohiko, c/o Shin-Etsu Chemical Co., Ltd., Usui-gun, Gunma-ken (JP)
(74) Representative: Armengaud Ainé, Alain

(57) **Abstract**

The invention provides a novel hair-care treatment composition such as shampoos and hair rinse compositions capable of imparting excellent smoothness in combing and pliableness and dampishness in touch feeling to the hair treated with the composition. The hair-care treatment composition is formulated, besides various base ingredients acceptable as the constituents of the respective hair-care treatment compositions, with a limited amount of a unique amino-modified diorganopolysiloxane having an amine equivalent in the range from 2000 to 100000 g/mole and represented by the general formula

A-SiR₂-O-(-SiR₂-O-)ₚ-(-SiRG-O-)_{q}-SiR₂-A,

in which the subscript p is a number of 10 to 20000, the subscript q is zero or a positive number not exceeding 100, R is a monovalent hydrocarbon group having 1 to 20 carbon atoms, e.g., methyl, G is an amino-substituted monovalent hydrocarbon group represented by the general formula

-R¹-(-NH-CH₂CH₂-)ₙ-NH₂,

R¹ being a divalent hydrocarbon group having 1 to 8 carbon atoms and the subscript n being 2 or 3, and A is R, OR, G or a hydroxyl group with the proviso that, when the subscript q is zero, at least one of the groups A in a molecule is G.

## Description

### BACKGROUND OF THE INVENTION

The present invention relates to a novel hair-care treatment composition or, more particularly, to a hair-care treatment composition such as shampoo compositions and hair rinse compositions capable of imparting greatly improved pliableness and smoothness in combing to the hair treated therewith.

As a consequence of the daily life, human hairs are unavoidably subject to mechanical damages by combing, brushing and shampooing and physical or chemical damages by exposure to sunlight, drying at high temperatures with a hair dryer and permanent-waving treatments to lose pliableness, smoothness and glossiness. With an object to mitigate the above mentioned adverse influences caused on the hair, it is an established practice that various kinds of hair-care treatment compositions are formulated with an oily ingredient including vegetable oils such as camellia oil and olive oil, animal oils such as lanolin and beef tallow and synthetic or mineral oils such as petrolatum and paraffins as a hair-protective oiling agent.

It is a discovery in rather recent years that improved glossiness, pliableness, slipperiness and dampishness can be imparted to the hair treated with a hair-care treatment composition containing an organopolysiloxane or so-called silicone of certain types. Based on this discovery, a great variety of silicone-containing hair-care treatment compositions are now under production and supplied to the market as a class of toiletry preparations.

For example, Japanese Patent Kokai 52-47923 discloses a hair-care treatment composition containing a dimethylpolysiloxane in combination with a diol compound or an aliphatic alcohol compound having a branched molecular structure. Japanese Patent Kokai 55-136214 proposes a hair conditioner composition containing an organopolysiloxane-polyoxyalkylene block copolymer and anhydrous or hydrous ethyl alcohol.

The hair-care treatment composition containing a dimethylpolysiloxane mentioned above, however, has a problem that the hair treated therewith is liable to accumulation of static electricity so that dust particles are readily deposited on the hair. Further, dimethylpolysiloxanes in general can not always give a fully stable water-base hair-care treatment composition such as shampoos, hair conditioners and hair rinses as an ingredient therein because of the strong hydrophobicity of the dimethylpolysiloxane. Further, the organopolysiloxane-polyoxyalkylene block copolymers are not fully effective for imparting good slipperiness to the hair treated with a hair-care treatment composition formulated therewith.

On the other hand, many proposals are made in Japanese Patent Kokai 55-66506, 57 58605, 58-74602, 58-210005, 59-130807, 59-176205, 60-36407, 60-56916, 61-78710, 62-12712, 63-51314, 63-88116, 63-218612, 63-275515, 63-307810, 63-307811, 1-172313, 1-190619, 1-203314, 2-160712 and 5-85918, Japanese Patent Publications 3-21523 and 3-33124 and elsewhere for the use of an amino group-containing organopolysiloxane or a derivative thereof, e.g., salts with an acid, amidation products and comodification products, as an ingredient in a hair-care treatment composition. The inventors of this invention also have disclosed, in Japanese Patent Kokai 9-194335, a hair-care treatment composition containing an amino-modified organopolysiloxane having specific polyoxyalkylene-based block units. The amino-containing substituent groups in the above mentioned modified organopolysiloxanes, however, are limited to monoaminoalkyl groups and diaminoalkyl groups such as N-(2-aminoethyl)-3-aminopropyl group and the like so that the advantageous effects to be obtained by the introduction of amino groups are far from satisfactory.

### SUMMARY OF THE INVENTION

The present invention accordingly has an object to provide a novel hair-care treatment composition or, in particular, a hair-care treatment composition such as shampoos and hair rinses containing a specific amino-modified organopolysiloxane capable of imparting greatly improved glossiness, pliableness, slipperiness and dampishness to the hair treated therewith.

Thus, the hair-care treatment composition provided by the present invention comprises, as a uniform blend:
(A) from 0.01 to 10% by weight of an amino-modified diorganopolysiloxane having an amine equivalent in the range from 2000 to 100000 g/mole and represented by the general formula

   A-SiR₂-O-(-SiR₂-O-)ₚ-(-SiRG-O-)_{q}-SiR₂-A, (I)

   in which the subscript p is a number in the range from 10 to 20000, the subscript q is zero or a positive number not exceeding 100, R is a monovalent hydrocarbon group having 1 to 20 carbon atoms, G is an amino-substituted monovalent hydrocarbon group represented by the general formula

   -R¹-(-NH-CH₂CH₂-)ₙ-NH₂, (II)

   R¹ being a divalent hydrocarbon group having 1 to 8 carbon atoms and the subscript n being 2 or 3, and A is R, OR, G or a hydroxyl group with the proviso that, when the subscript q in the general formula (I) is zero, at least either one of the groups A in a molecule is G; and
(B) the balance of ingredients acceptable as the base constituents of a hair-care treatment composition.

### DETAILED DESCRIPTION OF THE PREFERRED EMBODIMENTS

As is described above, the most characteristic feature of the inventive hair-care treatment composition is that the composition is formulated with a limited amount of a specific amino-modified diorganopolysiloxane represented by the general formula (I) as the characteristic ingredient.

In the general formula (I) representing the amino-modified diorganopolysiloxane as the component (A), the group denoted by R is, each independently from the others, a monovalent hydrocarbon group having 1 to 20 carbon atoms exemplified by alkyl groups such as methyl, ethyl, propyl, butyl, pentyl, hexyl, heptyl, octyl, decyl, dodecyl, tetradecyl and octadecyl groups, alkenyl groups such as vinyl and allyl groups, cycloalkyl groups such as cyclopentyl and cyclohexyl groups, aryl groups such as phenyl, tolyl and naphthyl groups and aralkyl groups such as 2-phenylethyl and 2-phenylpropyl groups as well as those substituted hydrocarbon groups obtained by replacing a part or all of the hydrogen atoms in the above named hydrocarbon groups with halogen atoms such as chlorine and fluorine atoms. Although the diorganopolysiloxane as the component (A) can contain two kinds or more of the above named hydrocarbon groups in combination in a molecule, it is preferable that all or at least 90% by moles of the groups denoted by R are methyl groups, the balance, if any, being, preferably, phenyl and/or vinyl groups.

The group denoted by G is an amino-substituted monovalent hydrocarbon group represented by the general formula (II) given above, in which R¹ is a divalent hydrocarbon group having 1 to 8 carbon atoms exemplified by alkylene groups such as methylene, ethylene, trimethylene and tetramethylene groups, of which trimethylene group is preferable. The subscript n in the general formula (II) is limited to 2 or 3 or, preferably, to 2 because a hair-care treatment composition containing a diorganopolysiloxane of the general formula (I) of which the subscript n in the general formula (II) is zero or 1 cannot impart full smoothness to the hair treated with the composition and an amino-substituted diorganopolysiloxane of the general formula (I) of which the subscript n in the general formula (II) is 4 or larger cannot be industrially prepared without difficulties.

The group denoted by A in the general formula (I) is R, OR, hydroxyl group or G. Examples of the group denoted by R are given above. The group OR is exemplified by methoxy, ethoxy, propoxy and butoxy groups, of which methoxy and ethoxy groups are particularly preferable in respect of the good adsorptivity of the amino-modified diorganopolysiloxane to the hair treated with the hair-care treatment composition.

The subscript p in the general formula (I) is an average number in the range from 10 to 20000 or, preferably, from 100 to 10000 or, more preferably, from 500 to 5000. When the value of the subscript p is too small, full smoothness cannot be imparted to the hair treated with the hair-care treatment composition formulated with such an amino-modified diorganopolysiloxane while, when the value of the subscript p is too large, the diorganopolysiloxane has an unduly high viscosity so that difficulties are encountered in the preparation of a uniform hair-care treatment composition containing the diorganopolysiloxane.

The subscript q in the general formula (I) is zero or a positive number not exceeding 100 or, preferably, zero or a positive number not exceeding 10 or, more preferably, zero or a positive number not exceeding 5. When the value of the subscript q is too large, fully improved smoothness cannot be imparted to the hair treated with the hair-care treatment composition formulated with such an amino-modified diorganopolysiloxane.

It is essential that, when the above defined subscript q in the general formula (I) is zero, at least either one of the two groups denoted by A terminating the molecular chain ends of the diorganopolysiloxane molecule must be the group denoted by G. This means that the amino-modified diorganopolysiloxane as the component (A) has, in a molecule, at least one amino-substituted hydrocarbon group represented by the general formula (II) as directly bonded to the silicon atom of the diorganopolysiloxane molecule.

As a consequence of the above described various requirements relative to the molecular structure, the amino-modified diorganopolysiloxane as the component (A) should have an amine equivalent in the range from 2000 to 100000 g/mole or, preferably, from 5000 to 80000 g/mole or, more preferably, from 10000 to 50000 g/mole. When the amine equivalent is too small or too large, fully improved smoothness cannot be imparted to the hair treated with the hair-care treatment composition formulated with such an amino-modified diorganopolysiloxane. The amine equivalent implied in the present invention is defined as the amount of the amino-modified diorganopolysiloxane in the unit of grams which provides a mole as a total of the amino groups -NH₂ and imino groups -NH-.

Some of the examples of the amino-modified diorganopolysiloxane suitable as the component (A) in the inventive hair-care treatment composition include those expressed by the following structural formulas (a) to (j), in which the symbols G² and G³ are the groups expressed by the formulas -C₃H₆-(-NH-CH₂CH₂-)₂-NH₂ and-C₃H₆-(-NH-CH₂CH₂-)₃--NH₂ with the subscript n in the general formula (II) equal to 2 or 3, respectively, Me is a methyl group and the subscripts p and q each have the same meaning as defined above:
(a) Me₃Si-O-(-SiMe₂-O-)ₚ-(-SiMeG²-O-)_{q}-SiMe₃;
(b) MeO-SiMe₂-O-(-SiMe₂-O-)ₚ-(-SiMeG²-O-)_{q}-SiMe₂-OMe;
(c) HO-SiMe₂-O-(-SiMe₂-O-)ₚ-(-SiMeG²-O-)_{q}-SiMe₂-OH;
(d) G²-SiMe₂-O-(-SiMe₂-O-)ₚ-(-SiMeG²-O-)_{q}-SiMe₂-G²;
(e) G²-SiMe₂-O-(-SiMe₂-O-)ₚ-SiMe₂-G²;
(f) Me₃Si-O-(-SiMe₂-O-)ₚ-(-SiMeG³-O-)_{q}-SiMe₃;
(g) MeO-SiMe₂-O-(-SiMe₂-O-)ₚ-(-SiMeG³-O-)_{q}-SiMe₂-OMe;
(h) HO-SiMe₂-O-(-SiMe₂-O-)ₚ-(-SiMeG³-O-)_{q}-SiMe₂-OH;
(i) G³-SiMe₂-O-(-SiMe₂-O-)ₚ-(-SiMeG³-O-)_{q}-SiMe₂-G³; and
(j) G³-SiMe₂-O-(-SiMe₂-O-)ₚ-SiMe₂-G³,
though not particularly limitative thereto.

The above described amino-modified diorganopolysiloxane as the component (A) which is the characteristic ingredient in the inventive hair-care treatment composition, is a known compound and can be prepared by a method undertaken in the prior art. For example, a siloxane rearrangement equilibration reaction is performed, in the presence of an alkaline compound as a catalyst such as alkali metal hydroxides, tetramethylammonium hydroxide and tetrabutylphosphonium hydroxide, in a mixture of a cyclic organosiloxane oligomer such as octamethyl cy-clotetrasiloxane, an amino-modified dialkoxy silane compound such as (MeO)₂MeSiG² and (MeO)₂MeSiG³, in which Me, G² and G³ each have the same meaning as defined above, or a hydrolysis product thereof and an organopolysiloxane or an organosilane compound including those to serve as a source material of the molecular chain terminal groups, such as hexamethyl disiloxane, α, ω -dihydroxy dimethylpolysiloxane and dimethoxy dimethyl silane.

The above described amino-modified diorganopolysiloxane can be used as such as the component (A) but can be used after conversion of the amino groups into the form of a salt by fully or partially neutralizing the same with an acid. The acid for neutralization of the amino groups can be either an organic acid such as formic, acetic, butyric, propionic, malonic and citric acids or an inorganic acid such as hydrochloric, sulfuric, nitric and phosphoric acids. An advantage is obtained by the conversion of the amino-modified diorganosiloxane into the form of a salt by the increase of the hydrophilicity or water-solublility thereof to facilitate compounding thereof with other ingredients including water and to improve the stability of a water-base hair-care treatment composition compounded therewith.

The amount of the above described amino-modified diorganopolysiloxane as the component (A) in the inventive hair-care treatment composition is in the range from 0.01 to 10% by weight or, preferably, from 0.1 to 5% by weight. When the amount of the component (A) is too small, the advantageous effects to be obtained by the addition of this component cannot be fully obtained as a matter of course. When the amount thereof is too large, on the other hand, the hair after treatment with a hair-care treatment composition containing an excessively large amount of the amino-modified diorganopolysiloxane would exhibit an unpleasant touch feeling of stickiness.

As is mentioned before, the hair-care treatment composition of the invention comprises from 0.01 to 10% by weight of the amino-modified diorganopolysiloxane as the component (A) and the balance of the component (B) which includes various ingredients acceptable as the base constituents of a hair-care treatment composition. Such acceptable ingredients in hair-care treatment compositions are well known by those skilled in the art of cosmetic and toiletry preparations and can be easily selected from the conventional formulations of the hair-care treatment composition of the respective types such as shampoos, hair rinses, hair conditioners and the like.

In the preparation of the inventive hair-care treatment composition, the amino-modified diorganopolysiloxane as the component (A) is compounded with other base ingredients including oleaginous materials and powders either as such or in the form of an aqueous emulsion or dispersion thereof obtained by emulsifying or dispersing the same in water in the presence of a surface active agent or in the form of a solution in water or in ethyl alcohol.

The hair-care treatment composition of the present invention in many cases is formulated with a surface active agent, which may be non-ionic, anionic, cationic or amphoteric depending on the particular types of the hair-care treatment compositions, together with waxes, vegetable oils, powder materials and the like. Examples of the surface active agent include fatty acid soaps, alkylbenzene sulfonates, alkyl sulfonates, alkyl ether sulfonates, monoglyceride sulfates, alkyl phosphates, methyl tauride and fatty acid alkanolamides.

When the inventive hair-care treatment composition is a shampoo composition not only for human hairs but also for animal hairs, the shampoo composition can be compounded, as one of the base ingredients, with an aliphatic higher alcohol such as lauryl and myristyl alcohols and derivatives thereof including ethoxylates and certain salts such as sodium lauryl sulfate, sodium lauryl ether sulfate, ammonium lauryl sulfate, triethanolamine lauryl sulfate, monoethanolamine lauryl sulfate and disodium lauryl ethoxysulfosuccinate as well as corresponding myristyl alcohol derivatives.

In short, the base ingredients as the component (B) in the inventive hair-care treatment composition to be combines with the amino-modified diorganopolysiloxane as the component (A) are selected from known materials depending on the types and forms of the hair-care treatment compositions which may be in the form of an aqueous solution or emulsion, solution in ethyl alcohol, aerosol, consolidated powder compact, paste, powder and others suitable for the particularly intended applications.

The inventive hair-care treatment composition includes, as classified depending on the application types, shampoos, hair rinses, hair conditioners, hair-set lotions, hair-styling lotions, pre-shampoo treatments, hair sprays, hair styling gels, hair dyes, hair bleaches, permanent-waving agents, hair tonics and others.

In the following, the hair-care treatment composition of the present invention is described in more detail by way of examples as preceded by the description of the procedure for the synthetic preparation of the amino-modified diorganopolysiloxanes as the component (A), although the scope of the present invention is never limited by these Examples in any way. The terms of "parts" and "%" appearing in the following description always refer to "parts by weight" and "% by weight", respectively, and the values of viscosity are all those obtained by the measurement at 25°C.

### Synthesis 1.

Into a separable flask of 500 ml capacity equipped with a stirrer, thermometer, reflux condenser and gas inlet tube were taken 122 g of 3-chloropropyl methyl dimethoxy silane which was heated with agitation under a stream of nitrogen gas up to a temperature of 70°C, where 206 g of diethylenetriamine were gradually added dropwise to the flask to form a reaction mixture. After completion of the dropwise addition of diethylenetriamine, the reaction mixture was agitated at 110°C for 3 hours to effect dehydrochlorination condensation between the silane compound and diethylenetriamine followed by distillation of the reaction mixture under reduced pressure to obtain a fraction having a boiling point of 132°C under a pressure of 2 mmHg which was identified to be an amino-substituted silane compound expressed by the structural formula of (MeO)₂MeSiG², in which each symbol has the same meaning as defined before, having a gas chromatographic purity of 97%.

In the next place, a 60 g portion of the above obtained silane compound was taken in the same separable flask of 500 ml capacity as used above, into which 13 g of water were gradually added dropwise under agitation to form a reaction mixture. After completion of the dropwise addition of water, the reaction mixture was agitated at room temperature for further 3 hours to complete hydrolysis of the silane compound. Thereafter, the reaction mixture was admixed with 30 g of toluene and the unreacted portion of water was removed by azeotropic dehydrating distillation followed by removal of toluene by distillation to give 54 g of a highly viscous hydrolysis-condensation product of the silane compound.

### Synthesis 2.

Into a separable flask of 1 liter capacity equipped with a stirrer, thermometer, reflux condenser and gas inlet tube were taken 700 g of octamethyl cyclotetrasiloxane, 6.1 g of dodecamethyl pentasiloxane and 3.2 g of the highly viscous hydrolysis-condensation product prepared in Synthesis 1 to form a mixture which was heated with agitation under a stream of nitrogen gas. When the temperature of the mixture in the flask had reached 110°C_{,} 0.4 g of tetrabutylphosphonium hydroxide was added to the mixture which was further agitated at the same temperature for 5 hours followed by further temperature increase up to 150°C where agitation of the reaction mixture was continued for additional 1 hour. Thereafter, the reaction mixture was subjected to stripping of volatile matters at 150°C under a reduced pressure of 3 mmHg to give 620 g of a clear and colorless liquid product having a viscosity of 6500 centipoise and an amine equivalent of 15000 g/mole. This liquid product could be assumed to be expressed by the average structural formula

Me₃Si-O-(-SiMe₂-O-)₆₀₀-(-SiMeG²-O-)₁-SiMe₃,

in which Me and G² each have the same meaning as defined before. this product is referred to as the organopolysiloxane-1 hereinafter.

### Synthesis 3.

The synthetic procedure was substantially the same as in Synthesis 2 described above excepting for a decrease of the amount of dodecamethyl pentasiloxane from 6.1 g to 1.0 g and a decrease of the amount of the highly viscous hydrolysis-condensation product obtained in Synthesis 1 from 3.2 g to 1.5 g to give 640 g of a clear and colorless product of gum-like consistency having an amine equivalent of 35000 g/mole. This gum-like product could be assumed to be expressed by the average structural formula

Me₃Si-O-(-SiMe₂-O-)₃₈₀₀-(-SiMeG²-O-)₃ -SiMe₃,

in which Me and G² each have the same meaning as defined before. This product is referred to as the organopolysiloxane-2 hereinafter.

### Example 1.

A shampoo composition was prepared by uniformly blending, in a propellor-blade mixing machine, the ingredients according to the following formulation.

| | |
|---|---|
| (1) polyoxyethylene (3 moles addition of ethyleneoxide) sodium lauryl sulfate | 16% |
| (2) lauryl sulfuric acid diethanolamide | 4% |
| (3) organopolysiloxane-1 | 2% |
| (4) propyleneglycol | 2% |
| (5) methyl p-oxybenzoate | 0.1% |
| (6) purified water | (balance to 100%) |

A shampooing test was undertaken by 10 panel members who shampooed their hair by using the above prepared shampoo composition followed by drying of the wet hair using a hair dryer directing their attention to the smoothness of the hair in combing. All of the panel members reported that their hair after shampooing and drying exhibited good smoothness in combing and gave a touch feeling of excellent pliableness as compared with the hair shampooed with a conventional commercial shampoo product.

### Example 2.

A hair rinse composition was prepared in the following manner. Firstly, 30 g of the organopolysiloxane-2 were mixed under agitation with 70 g of a dimethylpolysiloxane terminated at each molecular chain end with a trimethylsilyl group and having a viscosity of 100 centistokes to give a mixture, which is referred to as the organopolysiloxane-2 mixture hereinafter.

The formulation of the hair rinse composition prepared in this Example was as follows.

| | |
|---|---|
| (1) stearyl trimethyl ammonium chloride | 1% |
| (2) cetanol | 2% |
| (3) organopolysiloxane-2 mixture | 2% |
| (4) propyleneglycol | 5% |
| (5) hydroxyethyl cellulose | 1% |
| (6) methyl p-oxybenzoate | 0.1% |
| (7) purified water | (balance to 100%) |

In the preparation of the rinse composition, the ingredient (5) was dissolved in (7) at 70°C to give an aqueous solution to which the ingredients (1), (2), (3) and (4) kept at 70°C were added under agitation followed by cooling of the mixture to 50°C, where the ingredient (6) was added to the mixture, and further cooling to room temperature to give a hair rinse composition.

A rinsing test was undertaken by 10 panel members who, after shampooing of their hair by using a commercial shampoo product, applied the above prepared rinse composition to their wet hair followed by short rinsing with water and drying by using a hair dryer. All of the panel members reported that their hair after rinsing with the above prepared rinse composition and drying exhibited good smoothness in combing and gave a touch feeling of excellent pliableness as compared with the hair after rinsing by using a conventional commercial hair rinse product followed by drying.

### Comparative Example 1.

A comparative shampoo composition was prepared in just the same formulation as in Example 1 described above excepting for the replacement of the organopolysiloxane-1 with the same amount of another amino-modified dimethylpolysiloxane having a viscosity of 6200 centipoise and an amine equivalent of 15000 g/mole and expressed by the structural formula

Me₃Si-O-(-SiMe₂-O-)_{6 00}-(-SiMeG¹-O-)_{1.5} -SiMe₃,

in which Me is a methyl group and G¹ is a 3-(2-aminoethylamino)-propyl group of the formula -C₃H₆-NH-CH₂CH₂-NH₂.

A comparative shampooing test was undertaken in the same manner as in Example 1 excepting for the use of this comparative shampoo composition by 10 panel members who all reported that their hair after shampooing and drying was clearly inferior as compared with the hair after shampooing and drying in Example 1 relative to the smoothness in combing and pliableness in touch feeling.

### Comparative Example 2.

A comparative hair rinse composition was prepared in just the same formulation as in Example 2 excepting for the replacement of the organopolysiloxane-2 with the same amount of another amino-modified gum-like dimethylpolysiloxane having an amine equivalent of 35000 g/mole and expressed by the average structural formula

Me₃Si-O-(-SiMe₂-O-)₃₈₀₀-(-SiMeG⁰-O-)₈ -SiMe₃,

in which Me is a methyl group and G⁰ is a 3-aminopropyl group.

A comparative hair rinsing test was undertaken in the same manner as in Example 2 excepting for the use of this comparative hair rinsecomposition by 10 panel members who all reported that their hair after rinsing and drying was clearly inferior as compared with the rinsed and dried hair in Example 2 relative to the smoothness in combing and pliableness in touch feeling.

## Claims

1. A hair-care treatment composition which comprises, as a uniform blend:
(A) from 0.01 to 10% by weight of an amino-modified diorganopolysiloxane having an amine equivalent in the range from 2000 to 100000 g/mole and represented by the general formula
A-SiR₂-O-(-SiR₂-O-)ₚ-(-SiRG-O-)_{q}-SiR₂-A,
in which the subscript p is a number in the range from 10 to 20000, the subscript q is zero or a positive number not exceeding 100, R is a monovalent hydrocarbon group having 1 to 20 carbon atoms, G is an amino-substituted monovalent hydrocarbon group represented by the general formula
-R¹-(-NH-CH₂CH₂-)ₙ-NH₂,
R¹ being a divalent hydrocarbon group having 1 to 8 carbon atoms and the subscript n being 2 or 3, and A is R, OR, G or a hydroxyl group with the proviso that, when the subscript q is zero, at least either one of the groups A in a molecule is G; and
(B) the balance of ingredients acceptable as the base constituents of a hair-care treatment composition.

2. The hair-care treatment composition as claimed in claim 1 in which at least 90% by moles of the monovalent hydrocarbon groups denoted by R are methyl groups.

3. The hair-care treatment composition as claimed in claim 1 in which the divalent hydrocarbon group denoted by R¹ is an alkylene group.

4. The hair-care treatment composition as claimed in claim 3 in which the alkylene group denoted by R¹ is a trimethylene group.

5. The hair-care treatment composition as claimed in claim 1 in which the subscript n in the general formula representing the amino-substituted monovalent hydrocarbon group G is 2.

6. The hair-care treatment composition as claimed in claim 1 in which the subscript p in the general formula representing the amino-modified diorganopolysiloxane is a number in the range from 100 to 10000.

7. The hair-care treatment composition as claimed in claim 6 in which the subscript p in the general formula representing the amino-modified diorganopolysiloxane is a number in the range from 500 to 5000.

8. The hair-care treatment composition as claimed in claim 1 in which the subscript q in the general formula representing the amino-modified diorganopolysiloxane is zero or a positive number not exceeding 10.

9. The hair-care treatment composition as claimed in claim 8 in which the subscript q in the general formula representing the amino-modified diorganopolysiloxane is zero or a positive number not exceeding 5.

10. The hair-care treatment composition as claimed in claim 1 in which the amine equivalent of the amino-modified diorganopolysiloxane is in the range from 5000 to 80000 g/mole,

11. The hair-care treatment composition as claimed in claim 10 in which the amine equivalent of the amino-modified diorganopolysiloxane is in the range from 10000 to 50000 g/mole,

12. The hair-care treatment composition as claimed in claim 1 in which the amino-modified diorganopolysiloxane is in the form of a salt as at least partially neutralized with an acid.

13. The hair-care treatment composition as claimed in claim 10 in which the amount of the amino-modified diorganopolysiloxane as the component (A) is in the range from 0.1 to 5% by weight of the composition.
